(19) Europäisches Patentamt
European Patent Office
Office européen des brevets

(11) Numéro de publication: **0 146 455 B1**

(12) # FASCICULE DE BREVET EUROPEEN

(45) Date de publication de fascicule du brevet: **15.04.92**

(51) Int. Cl.[5]: **C08B 37/02**, A61K 31/73

(21) Numéro de dépôt: **84402466.1**

(22) Date de dépôt: **30.11.84**

Demande divisionnaire 90122614.2 déposée le 30/11/84.

(54) **Dérivés du dextrane dotés, notamment, de propriétés anticoagulantes et de propriétés anticomplémentaires, leur préparation et leurs applications biologiques.**

(30) Priorité: **30.11.83 FR 8319110**

(43) Date de publication de la demande:
**26.06.85 Bulletin 85/26**

(45) Mention de la délivrance du brevet:
**15.04.92 Bulletin 92/16**

(84) Etats contractants désignés:
**AT BE CH DE FR GB IT LI LU NL SE**

(56) Documents cités:

**BIOMATERIALS, vol. 3, octobre 1982, pages 221-224, Butterworth & Co., Ltd.; M. MAUZAC et al.: "Antithrombic activity of some polysaccharide resins"**

(73) Titulaire: **CHOAY S.A.**
**40 Avenue George V**
**F-75008 Paris(FR)**

(72) Inventeur: **Mauzac, Monique**
**49, Avenue de la Plage La Hume**
**F-33470 Gujan Mestras(FR)**
Inventeur: **Jozefonvicz, Jacqueline**
**65, 2ème Avenue**
**F-60260 La Morlaye(FR)**
Inventeur: **Jozefonvicz, Marcel**
**65, 2ème Avenue**
**F-60260 La Morlaye(FR)**

(74) Mandataire: **Polus, Camille et al**
**c/o Cabinet Lavoix 2, Place d'Estienne d'Orves**
**F-75441 Paris Cedex 09(FR)**

**EP 0 146 455 B1**

Il est rappelé que: Dans un délai de neuf mois à compter de la date de publication de la mention de la délivrance du brevet européen, toute personne peut faire opposition au brevet européen délivré, auprès de l'Office européen des brevets. L'opposition doit être formée par écrit et motivée. Elle n'est réputée formée qu'après paiement de la taxe d'opposition (art. 99(1) Convention sur le brevet européen).

## Description

L'invention est relative à des dérivés du dextrane dotés, notamment, de propriétés anticoagulantes et de propriétés anticomplémentaires, à leur préparation et à leurs applications biologiques.

On sait que l'héparine est largement utilisée comme médicament antithrombotique en raison de ses propriétés anticoagulantes élevées.

Des travaux récents ont également montré son action vis-à-vis de l'inactivation du système complementaire, c'est-à-dire l'ensemble des protéines plasmatiques jouant un rôle essentiel dans la défense immunitaire de l'organisme.

L'hétérogénéité de ses chaînes, notamment, en ce qui concerne leur composition et leur longueur, rend toutefois difficile l'étude des structures responsables de ses propriétés.

De nombreux travaux ont été entrepris pour élaborer des produits présentant au moins certaines des propriétés spécifiques de l'héparine, mais de structure bien définie pouvant ainsi servir de modèle pour l'étude des mécanismes correspondants mis en jeu.

Certains des co-inventeurs de la présente invention ont ainsi mis au point et décrit (brevets français No 2 461 724 et Européen No 80 401053.6) des produits doués de propriétés anticoagulantes constitués par des polymères comportant des groupes :

$-SO_3R_1$, $-R_3SO_3R_1$, $-SO_2R_2$, $-R_3-SO_2-R_2$ et $-CH_2-CO-NH-CHR-COOH$-dans lesquels :

$R_1$ est un atome d'hydrogène ou d'un métal physiologiquement compatible,

$R_2$ est un reste d'acide aminé lié au pont $-SO_2-$ par la fonction amine,

$R_3$ est un groupe $-CH_2-CO-NH-R_4$ dans lequel $R_4$ représente un radical alcoyle, aryle ou alcoylaryle, ou

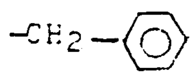

substitués ou non

et

R représente la chaîne latérale d'un acide aminé.

Les travaux des inventeurs dans ce domaine les ont amenés à étudier un polysaccharide déjà connu, à savoir, le dextrane.

Le dextrane est un polyglucoside de poids moléculaire d'environ 5000 à plusieurs millions de daltons, formé d'unités glucosyle A, liées par des enchaînements essentiellement 1-6, de formule :

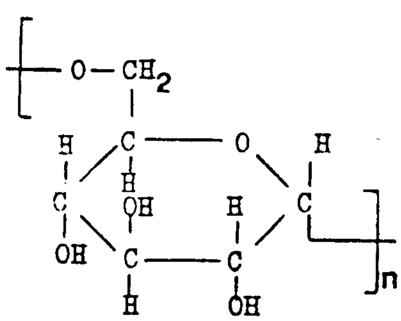

Pour les applications en thérapeutique, on utilise des dextranes de poids moléculaire généralement inférieur à environ 100 000.

Ce produit est utilisé, notamment, comme substitut du plasma sanguin ou du moins comme plasma expandeur, ou rétablisseur de volume. Ces utilisations peuvent entraîner cependant, dans certains, cas des chocs immunitaires.

Les travaux effectués ont montré que la présence simultanée de certains groupes de substitution, selon des proportions déterminées, sur des chaînes de dextrane dépourvues d'acide aminés leur conférait des propriétés biologiques de grand intérêt et une tolérance élevée.

L'invention a donc pour but de fournir des dérivés du dextrane possédant au moins certaines des propriétés biologiques de l'héparine et utilisables comme biomatériaux grâce à leur tolérance élevée.

Elle vise également à fournir un procédé de préparation de ces dérivés de mise en oeuvre aisée.

Elle vise également à fournir des biomatériaux solubles et des médicaments ou vecteurs de médicaments de grande efficacité à base de ces dérivés présentant l'avantage d'être obtenus par voie de synthèse.

Les dérivés de dextrane de l'invention sont caractérisés en ce qu'ils sont solubles, qu'ils possèdent un poids moléculaire supérieur à environ 5000 daltons, et qu'ils comportent de manière statistique,

- au moins 35% environ et plus spécialement 40% de motifs B constitués de motifs osides substitués par des radicaux possédant une fonction carboxyle répondant à la structure $-O-(CH_2)_n-R-COO^-$ dans laquelle R représente une simple liaison ou un groupe $-CO-NH-(CH_2)_{n'}-$, n est un nombre de 1 à 10 et n'est un nombre de 1 à 7, et
- au moins 3% environ de motifs D, c'est-à-dire des motifs constitués de motifs osidiques de type A, substitués par une chaîne comportant un groupe de structure :

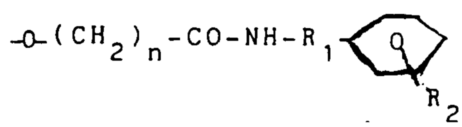

dans laquelle :

- $R_1$ représente une simple liaison, un groupe $-CH_2-$, ou un groupe

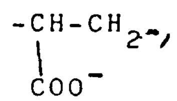

$R_2$ représente un anion d'un sel minéral ou organique physiologiquement tolérable, plus spécialement un groupe $SO_3^-$, et n est tel que défini ci-dessus.

La fonctionnalisation du support dextrane à l'aide des chaînes de substitution définies ci-dessus confère de manière avantageuse à ce dernier une activité anticoagulante. Cette activité, bien que plus faible que celle de l'héparine, présente un grand intérêt pour les applications biologiques de ces dérivés.

Ces produits apparaissent, en outre, dotés d'une activité anticomplémentaire du même ordre de grandeur que celle de l'héparine.

On observera que ces effets avantageux sont obtenus par la présence simultanée (1) de groupes carboxyle sur les motifs B et (2) de chaînes de substitutions, sur les motifs D, comportant des groupes aryle substitués par un anion d'un sel minéral ou organique et plus spécialement des groupes arylsulfonates et reliés à la chaîne de dextrane par un bras comportant un groupe amide.

Selon une disposition avantageuse de l'invention, les dérivés de dextrane renferment en outre des motifs C substitués par des radicaux de structure

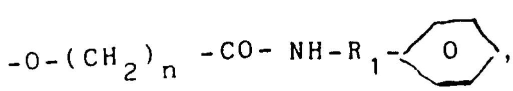

dans laquelle $R_1$ et n sont tels que définis ci-dessus.

L'ensemble des motifs A non substitués du dextrane et des motifs C ci-dessus représentent au plus 60% du nombre total de motifs.

Une famille préférée de dérivés de dextrane selon l'invention comprend des motifs B substitués par une chaîne $-O-CH_2-COO^-$.

Dans une autre famille de l'invention, les motifs B sont substitués par des groupes carboxyéthyle, carboxypropyle ou carboxybutyle $-O-(CH_2)_n-COO^-$, n étant égal respectivement à 2,3 ou 4.

Selon une autre famille préférée, les motifs B sont substitués par des groupes $-O-(CH_2)_n-CO-NH-(CH_2)_{n'}-COO^-$.

Les dérivés dans lesquels n' est égal à 4,5 ou 7 comprennent un radical $-NH-(CH_2)_{n'}-COO^-$ correspondant respectivement à un groupe acide valérique, acide aminocaproïque et acide aminocaprylique.

Un groupe préféré de dérivés de dextrane de l'invention renferme, en même temps que les motifs B de l'une des familles définies ci-dessus des motifs D substitués par une chaîne de structure

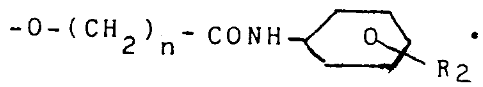

Dans un autre groupe préféré, les chaînes de dextrane renferment en plus des motifs B ci-dessus, des motifs D substitués par une chaîne de structure

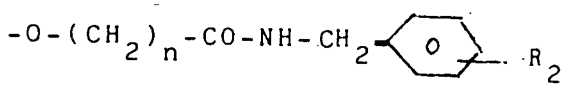

Dans un autre groupe encore, les motifs D sont substitués par une chaîne de structure

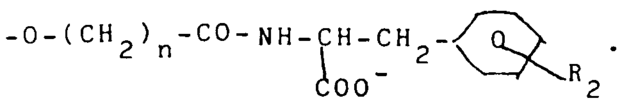

Dans ces différents groupes, $R_2$ est tel que défini ci-dessus et représente avantageusement un groupe $-SO_3^-$ et n est un nombre de 1 à 4, de préférence égal à 1.

D'une manière générale, les substitutions des motifs B, C, et D occupent essentiellement la position 2 du motif glucosyle de base. Les dérivés dans lesquels ces substitutions occupent d'autres positions ainsi qu'éventuellement la position 2, entrent cependant dans le cadre de l'invention.

De même, l'invention vise également les dérivés dans lesquels une partie des groupes -OH des glucosyles se présentent sous forme $-OR_2$.

En préparant toute une gamme de dérivés, les inventeurs ont pu mettre en évidence que l'activité anticoagulante devient très faible lorsque le taux des motifs B est inférieur à 35% environ. Il en est de même en l'absence totale de motifs D.

D'une manière générale, pour un taux de motifs B de l'ordre de 40 à 50%, on observe une augmentation de l'activité anticoagulante avec celle du nombre de motifs D et une activité anticomplémentaire sensiblement égale à celle de l'héparine.

Des dérivés de ce type, renfermant 9-12% environ de motifs D, de prérérence juseu'à 20% environ, présentent ainsi une activité antithrombique pouvant atteindre des valeurs de l'ordre de 300 à 400u T/mg. Des produits avantageux correspondants possèdent des poids moléculaires élevés supérieurs à 40000 et même à 60 ou 80000 environ.Dans ces produits le taux de motifs C peut être nul.

L'activité antithrombique est plus faible de l'ordre de 1 à 5uT/mg et l'activité anticomplémentaire toujours plus élevée de l'ordre de 1 à $10\mu g/10^7$ EAC 4b3bBbP chez les dérivés possédant un pourcentage plus faible de motifs D d'environ 4%, le taux de motifs B étant de 40 à 50%. Chez ces dérivés, le poids moléculaire est plus faible, d'environ 10000. Les activités antithrombiques et anticomplémentaires sont mesurées selon les méthodes rapportées dans les exemples.

Une catégorie particulièrement préférée de dérivés de l'invention comprend les dérivés de dextrane comportant les motifs A,B,C, et D suivants :

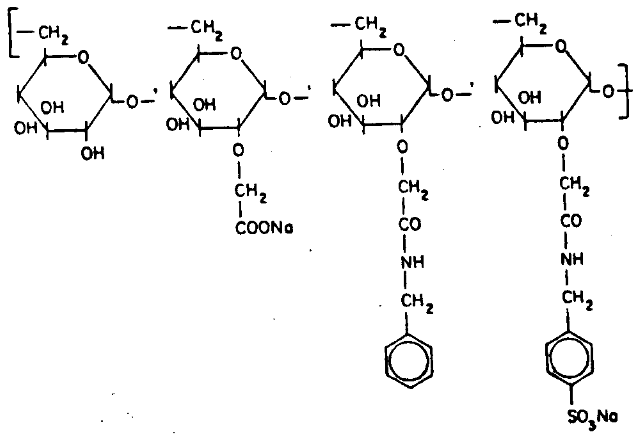

A B C D

selon les proportions données ci-dessus.

L'invention vise également un procédé de préparation des dérivés de dextrane définis ci-dessus.

Selon ce procédé, on met en oeuvre une chaîne de dextrane formée de motifs A non substitués et on élabore successivement les motifs B, C et D, ces motifs étant chacun obtenu à partir de celui préparé auparavant selon l'ordre indiqué.

L'élaboration de ces différents motifs sur la chaîne de dextrane comprend avantageusement les étapes suivantes, à savoir :

- la réaction d'un dextrane avec un dérivé de formule :

  $X(CH_2)_n\text{-}R\text{-}COOH$

  dans laquelle X représente un groupe réactif capable d'établir une liaison de glucosylation avec un groupe -OH d'un motif glucosyle, ce qui conduit à la formation de motifs B;
- la réaction du dextrane renfermant les motifs A et B avec un dérivé de formule

  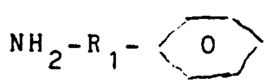

  dans laquelle $R_1$ est tel que défini ci-dessus, afin d'obtenir la fixation par un pont amide du groupe aryle substitué au radical provenant des chaînes de substitution des motifs B, ce qui permet d'introduire des motifs C sur la chaîne,
- l'introduction de groupes anioniques sur les groupes aryle de motif C, plus spécialemen- de groupes $SO_3^-$ , pour obtenir les motifs D,
- le fractionnement éventuel des dérivés de dextrane afin d'éliminer les dérivés présentant un poids moléculaire inférieur à 5000.

La séparation des dextranes peutêtre effectuée en premier, suivie des étapes évoquées ci-dessus.

Chacune des étapes ci-dessus est éventuellement répétée jusqu'à l'obtention du taux désiré de motifs sur la chaîne.

Pour l'élaboration des motifs B, on fait avantageusement réagir avec le dextrane un dérivé réactif tel qu'un halogénure, plus spécialement pour des raisons de disponibilité, un chlorure.

La réaction de glucosylation est effectuée en milieu basique dans des conditions permettant d'éviter la dégradation de la chaîne de dextrane sensible à l'hydrolyse.

A cet égard, le mélange réactionnel basique renfermant le dextrane est porté à une température de l'ordre de 0 ° C,ou inférieure, notamment de -4 ° C à + 5 ° C.

Après addition du dérivé réactif, le mélange est porté à une température supérieure à l'ambiante, pouvant atteindre 70 ° C environ.

De préférence, on procède selon un gradient de température, en faisant croître progressivement la température de l'ambiante à 55 ° C environ. Le réglage de la température permet de faire varier le rendement de substitution pratiquement à volonté. Il est ainsi possible d'obtenir aussi bien des taux de carboxyle de 40 à 60% que des taux atteignant 80% en une étape et pratiquement 100% en deux étapes

et même les dépassant.

Toute une gamme de produits répondant aux propriétés souhaitées est ainsi accessible.

Cette modulation présente un intérêt étant donné que lorsqu'on souhaite disposer de produits dotés de propriétés anticoagulantes élevées, il est préférable de disposer d'un taux plus élevé de motifs D,alors que ce taux doit être plus élevé pour augmenter l'activité anticomplémentaire des dérivés.

Le rapport de la concentration en dérivé réactif à celle du dextrane est avantageusement de 1,5 à 3,5 environ.

Afin de permettre la récupération des produits par précipitation, le pH est abaissé jusqu'à un pH neutre.

Les produits sont précipités à l'aide d'un solvant notamment un solvant alcoolique tel que le méthanol.

Lorsque la chaîne R dans les motifs B représente un groupe -CO-NH-$(CH_2)_{n'}$, on obtient avantageusement les produits à partir des motifs B substitués par une chaîne -O- $(CH_2)_n$-$COO^-$ par réaction avec les acides aminés correspondants.

L'étape d'élaboration des motifs C comprend le couplage des dérivés de formule

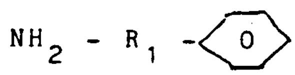

avec la chaîne de substitution des motifs B.

On utilise avantageusement un agent de couplage en milieu acide, à température ambiante.

Il s'agit d'agents de couplage tels que ceux mentionnés dans l'ouvrage Réactifs IBF "Practical guide for use in affinity chromatography", 1979, p.34-37, notamment de la N-éthoxycarbonyl-2-éthoxy-1,2-dihydrocholine (EEDQ), le carbodiimide ou le N-isobutoxy carbonyl -2 isobutoxy- 1,2 dihydroquinoline (I.I.D.Q).

Selon une variante permettant de réaliser la réaction avec un rendement élevé, à savoir, environ au moins 10% de substitution, on prépare l'anhydride mixte du dextrane en faisant réagir un chloroformate d'alcoyle (d'éthyle ou d'isobutyle notamment) et un dérivé capable de former un chlorhydrate avec l'acide chlorhydrique libéré tel que la triéthylamine, la N-méthylmorpholine ou analogue. Cette étape d'activation est réalisée de préférence à une température inférieure à 0°C et même de -15°C environ. Cette réaction s'effectue très rapidement en quelques minutes puis on réalise le couplage à basse température.

Le rapport de la concentration en dérivé à coupler à celle du dextrane substitué est avantageusement de l'ordre de 2 et celui du chloroformate par rapport au dextrane environ de 1.

On fait ensuite réagir les chaînes de dextrane formées renfermant des motifs B avec un dérivé réactif contenant $R_2$ et permettant de fixer $R_2$ sur le noyau aryle des chaînes de B.

La substitution par des groupes $SO_3^-$ est avantageusement réalisée à l'aide d'acide chlorosulfonique, dans des conditions diluées afin d'éviter la dégradation de la chaîne de dextrane. Cette étape est réalisée en phase hétérogène et le produit est récupéré lavé et séché.

Au cours de cette étape, il est possible de substituer jusqu'à environ la moitié des cycles aromatiques des motifs C.

L'opération est renouvellée si nécessaire jusqu'à l'obtention du taux de substitution désiré.

En vue de ses applications biologiques, le dextrane substitué ainsi obtenu est lavé puis conservé sous forme lyophilisée.

Grâce à ce procédé, il est possible de préparer par voie de synthèse, une gamme de dérivés de dextrane présentant l'avantage d'être solubles, de proportions en motifs de substitutions très variables, ces proportions pouvant être choisies en fonction des propriétés recherchées.

L'activité biologique des dérivés de dextrane définis ci-dessus a été étudiée à différents niveaux notamment sur certaines protéines de la coagulation, en particulier, la thrombine, et sur le système du complément.

Les résultats obtenus montrent que l'activité de ces dérivés vis-à-vis de la thrombine dépend du pourcentage de motifs B et D.

Cette activité apparaît pour un taux de motifs B supérieur à 35%, plus spécialement à 40% et croît avec le pourcentage de motifs D. Cette activité semble donc résulter d'un effet coopératif entre les chaînes de substitution de B et de D.

L'examen des fractions isomoléculaires de dérivés de l'invention a montré un accroissement très net de l'activité d'inhibition de la thrombine avecla masse moléculaire.

L'étude de leur action sur les protéines plasmatiques du système complémentaire a mis en évidence un fort pouvoir d inhibition vis-à-vis de de l'action de la $C_3$ convertase alterne, complexe enzymatique capable de priver la protéine $C_3$ de la voie alternedu système complémentaire.

En présence du sang, ces produits sont ainsi capables de conduire, autant que l'héparine, à une

diminution de l'hémolyse et à une décroissance de la réponse inflammatoire de l'organisme.

L'intérêt de ces dérivés est encore accru en raison de leur bonne tolérance.

Les tests de toxicité ont été effectués sur des souris d'environ 20 g par injection intraveineuse de 0,5ml par souris, d'une solution de 40 mg/ml du dextrane modifié conforme à la présente invention dans du soluté NaCl isotonique. Ces tests ont démontré la totale inocuité des produits conformes à la présente ivention : aucune réaction ni pendant l'injection, ni pendant 14 jours suivant l'injection, n'a été constatée.

Compte-tenu de leurs propriétés, ces dérivés sont particulièrement appropriés pour l'utilisation en tant que substituts de plasma sanguin plus spécialement comme expandeurs avec l'avantage par rapport aux dextranes de diminuer les risques d'hypersensibilités observées dans certains cas.

L'invention vise donc également des biomatériaux utilisables comme substituts de plasma sanguin et expandeurs à base des dérivés de dextrane ci-dessus, en solution de 5 à 15% environ, notamment 10% environ dans un milieu salin. Ces expandeurs sont utilisés, par exemple, à raison d'environ 1,5 à 2 l/jour.

Comme montré par les exemples, les dérivés de dextrane de l'invention, en particulier, ceux de poids moléculaire élevé, supérieur à 40 000 environ, possèdent une activité antithrombique a de l'ordre de 300 à 400 u T/mg ou plus.

De tels produits, comme indiqué ci-dessus, comportent avantageusement de 40 à 50% de motifs B environ et 9-12% et même jusqu'à 20% environ de motifs C Des produits préférés possèdent un poids moléculaire supérieur à 60 000 et même à 80 000.

L'invention concerne donc des compositions pharmaceutiques comprenant les dérivés de dextrane de l'invention et plus spécialement des compositions présentant une activité anticoagulante comportant, en tant que principe actif, une quantité efficace d'au moins un dérivé tel que défini ci-dessus avec les proportions préférées indiquées. Une composition préférée renferme les dérivés de dextrane de l'exemple 1, avantageusement selon les proportions qui précèdent pour les motifs B et D, la teneur en C pouvant être nulle.

Ces compositions renferment un véhicule pharmaceutique approprié pour l'administration par voie orale, rectale, sous forme de pommades, ou injectable.

Des solutions injectables contiennent de 10 à 50 mg de dextrane par prise. L'injection peut être effectuée par voie sous-cutanée, intraveineuse ou par perfusion et répétée 2 à 3 fois par jour selon l'état du patient et les résultats des essais biologiques, comme effectué dans les traitements usuels hépariniques.

Les dérivés de l'invention constituent également des produits intermédiaires de synthèse pour l'élaboration de vecteurs de médicaments. Ils sont, en effet, utilisables comme support pour véhiculer des substances actives de divers types.

L'invention est exposée plus en détail dans les exemples qui suivent relatifs à la préparation de dérivés de dextranes et à l'étude de leurs activités biologiques.

EXEMPLE 1 :

Préparation de dérivés de dextrane comportant les motifs A, B, C et D suivants :

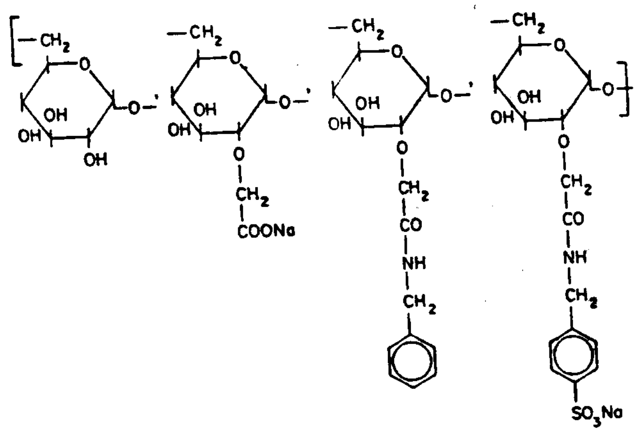

A B C D

On soumet un échantillon de dextrane d'un poids moléculaire supérieur à 8000, la séparation ayant été effectuée par chromatographie liquide sous basse pression, aux étapes 1 à 5 suivantes :

1) : carboxyméthylation à l'aide d'acide monochloroacétique -

2) : fixation de benzylamine par couplage de ce dérivé -

3) : sulfonation des noyaux aromatiques de la benzylamine -

4) : lavages et conditionnement.

1ère étape : carboxyméthylation des dextranes

Cette étape est inspirée par les travaux de F. ANTONINI & Coll. (Giorn. Biochi. 14, 88, 1965).

Dans un ballon de 1 litre muni d'un système d'agitation et immergé aux deux tiers dans un bain [glace + sel (-4 ° C], on dissout 48,6 g de dextrane (0,30 Mole) dans 400 ml de soude 6 (2,4 Moles). On laisse sous agitation à -4 ° C pendant 20 minutes.

Dans le ballon, on introduit petit à petit (durée d'introduction 10 minutes) 100 g de $ClCH_2COOH$ (1,05 Moles).

On porte la température à 70 ° C et on maintient à cette température et sous agitation pendant 20 minutes.

On refroidit en immergeant le ballon aux deux tiers dans un bain de glace.

Le pH qui présente une valeur voisine de 11 est ajusté à 7 par addition d'acide acétique.

On précipite le produit à l'aide de 3 litres de méthanol puis on le lave au méthanol et on le sèche dans une étuve sous vide à 40 ° C environ.

Par cette méthode, on substitue environ 30% des cycles dextranes, sans dégradation des chaînes macromoléculaires. Les substitutions plus importantes sont obtenues en répétant plusieurs fois (n fois) cette opération selon le Tableau I ci-après :

TABLEAU I

| n (nombre de carboxyméthylation) | % de motifs dextranes carboxyméthylés |
|---|---|
| 1 | 30-32 |
| 2 | 45-50 |
| 3 | 60-65 |
| 4 | 75-80 |
| 5 | 90-98 |

2ème étape : Fixation de la benzylamine

Cette étape est inspirée par les travaux de P.V. SUNDARAM [Biochem.Biophys.Res.Comm. 61, 717,1974]

Dans un ballon de 2 litres, on dissout sous agitation à température ambiante 40 g de carboxyméthyl-dextrane à 90% de motifs dextranes carboxyméthylés (préparé au cours de la 1ère étape) dans 290ml

d'eau.

On ajuste le pH à environ 3,5 par de l'acide chlorhydrique 1N.

On verse 89 g (0,360 Mole) de N-éthoxycarbonyl-2-éthoxy-1,2-dihydroquinoline (E.E.D.Q.) de formule :

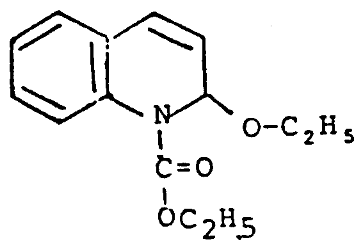

dissous dans 710 ml d'éthanol absolu.

On maintient sous agitation pendant une demiheure.

On ajoute 40 ml de benzylamine (0,36 Mole) et on laisse sous agitation une nuit.

Après évaporation du mélange presque à sec, on précipite le dextrane à l'aide de 2 litres de méthanol, on lave au méthanol et on sèche à l'étuve sous vide à 40 ° C.

3ème étape : Sulfonation des noyaux aromatiques de la benzylamine -

12 g de produit obtenus au cours de la 2ème étape et comportant 1 mMole/g de benzylamine sont dispersés sous agitation dans 240 ml de chlorure de méthylène.

On ajoute lentement 2,4 ml d'acide chlorosulfonique (0,036Mole), on laisse le mélange réactionnel sous agitation une nuit, on filtre, on lave à l'éthanol puis on sèche à l'étuve sous vide à 40 ° C.

Par cette méthode, en substitue jusqu'à environ la moitié des cycles aromatiques de la benzylamine.

Pour obtenir les taux de sulfonation désirés, on renouvelle l'opération plusieurs fois.

4ème étape : Lavages et conditionnement -

Le produit obtenu en fin de la 3ème étape est dissous dans une solution aqueuse de soude et le pH est maintenu à 8 pendant 2 heures.

Il est ensuite lavé à l'eau, équilibré à pH 7,35 par du tampon de Michaelis, puis relavé à l'eau. Toutes les opérations sont effectuées en utilisant la méthode d'ultrafiltration (membranes semi-perméables à seuil de coupure adapté).

Le produit est finalement lyophilisé.

EXEMPLE 2 :

Variante de réalisation de l'étape de carboxyméthylation et de couplage de la benzylamine.

. carboxyméthylation :

En variante, on opère comme suit :

On dissout 30 g (0,185 mole) de dextrane de poids moléculaire de 40 000 environ dans 246 ml de soude 6N (1,48 mole) et on agite pendant 20 minutes à cette température.

On introduit ensuite lentement dans le ballon 61,2 g de $ClCH_2COOH$ (0,647 mole), en maintenant la température à 20 ° C.

Le système est ensuite chauffé pour atteindre 55 ° C au bout de 20 minutes.

La réaction est ainsi maintenue pendant un temps variable $t_2$ (de 0 à 60 minutes) selon le degré de substitution désiré (25% à 85% de carboxyméthyl-dextrane).

Il est également possible de contrôler le rendement de cette carboxyméthylation en fixant le temps $t_2$ à 40 minutes et en jouant sur le rapport $R_2$ de la quantité d'acide monochloacétique à la quantité de motif dextrane selon $1 \leqq R \leqq 3,5$.

. couplage de la benzylamine .

Dans un réacteur double enveloppe (6,17mmoles) relié à un cryostat, 1 g de carboxymétyl dextrane de poids moléculaire de l'ordre de 40 000 (98% CMD) est dissous dans 8 ml d'eau et on acidifie le milieu à pH 3,5 environ avec CHl concentré. On ajoute alors très lentement 16 ml de diméthylformamide tout en maintenant le pH vers 3,5.

La solution est alors portée à -15 ° C.

On ajoute ensuite un volume $V_1$ (0,44ml) de N-méthylmorpholine et un volume $V_2$ (0,52ml) d'isobutylchloroformate.

Une minute après ce dernier ajout, on introduit un volume $V_3$ (0,49ml) de benzylamine.

La réaction est maintenue sous agitation pendant un temps $t_1$ (5 à 60 mn) à -15 ° C, puis la température est amenée à 20 ° C et on agite pendant 1 heure.

Le produit est alors tiré sous vide puis précipité dans 500 ml de méthanol, filtré sur fritte puis remis dans l'eau pour être ultrafiltré sur membrane calibrée.

On obtient ainsi des taux de benzylamine fixée variant entre 1% et 15% en une étape.

## EXEMPLE 3

En procédant comme indiqué dans l'exemple 1 ou 2 ci-dessus, on a préparé une série de produits substitués de façon variée. Ces produits sont rapportés dans le tableau 1 suivant avec une indication de leur composition et de leur masse moléculaire moyenne en nombre.

TABLEAU 1

| Référence | $\overline{Mn}$ | | Composition | | |
|---|---|---|---|---|---|
| | Valeur théorique | Mesure par tonométrie | B % ± 1 | C % ± 1 | D % ± 1 |
| 1 (Dextrane) | 4900 | | 0 | 0 | 0 |
| 2 C.M. Dextrane | 5600 | 5300 | 30 | 0 | 0 |
| 3 C.M. Dextrane | 7200 | 7000 | 95 | 0 | 0 |
| 4 | | | 51 | 14 | 0 |
| 5 | 6714 | 7100 | 37 | 21 | 0 |
| 6 | | | 37 | 11 | 9 |
| 7 | 6721 | 6930 | 37 | 15 | 5 |
| 8 | | | 37 | 15 | 6 |
| 9 | | | 70 | 7 | 1 |
| 10 | | | 71 | 7 | 1 |
| 11 | | | 47 | 1 | 3 |
| 12 | 6628 | 6670 | 40 | 12 | 3 |
| 13 | | | 40,5 | 4 | 4 |
| 14 | | | 40,5 | 4 | 10 |
| 15 | | | 43 | 0 | 15 |
| 16 | | | 45 | 0 | 4 |
| 17 | | | 60 | 14 | 5 |
| 18 | | | 76 | 0 | 4,5 |
| 19 | | | 58 | 5 | 6 |
| 20 | | | 67 | 0 | 5 |
| 21 | 6140 | 5980 | 45 | 0 | 5 |
| 22 | | | 50 | 3 | 10 |
| 23 | | | 43 | 4 | 11 |
| 24 | | | 49 | 1 | 12 |
| 25 | | | 68 | 4 | 12 |
| 26 | | | 71,5 | 0 | 10 |
| 27 | | | 75 | 0 | 14 |
| 28 | | | 45 | 0 | 14 |

## EXEMPLE 4 :

Etude du temps de thrombine, du temps de reptilase et de l'activité anticoagulante de dérivés de l'invention -

4.1 : Temps de thrombine et temps de reptilase.

. méthode

On mesure le temps de thrombine (TT) et le temps de reptilase (TR).

Le temps de thrombine correspond au temps de formation d'un caillot après addition de thrombine dans un échantillon de plasma ou une solution de fibrinogène.

Le temps de reptilase permet de contrôler que la capacité de transformation du fibrinogène en fibrine n'est pas modifiée par la présence du dérivé de dextrane.

Ces mesures sont déterminées automatiquement à 37°C à l'aide d'un coagulomètre Dade KC1, en opèrant comme suit :

0,2 ml de plasma pauvre en plaquettes (PPP) ou de fibrinogène (6g/l) est incubé à 37°C avec 0,1ml de tampon de Michaelis contenant selon une concentration appropriée le dérivé de dextrane dissous.

La durée d'incubation est de 5mn.

On ajoute 0,1 ml de solution de thrombine (dans un tampon de Michaelis) ou de reptilase (dans de l'eau distilée) et on mesure le temps d'apparition du caillot (TT ou TR).

Le temps témoin est déterminé avec un volume de tampon exempt de dérivé de dextrane.

L'évolution du TT sur plasma en fonction de la concentration du polymère apparaît parallèle à celle observée avec l'héparine comme le montre la figure 1. Sur cette figure on a indiqué la variation du TT en fonction(1) d'un dérivé renfermant 14% de motif D et 45% de motif B (courbe ●), (2) d'une héparine (courbe ♦).

L'héparine utilisée possède une activité USP de 173 ui/mg et un poids moléculaire de 10 700 daltons.

. résultats

Sur le tableau 2, on a rapporté les temps de thrombine sur plasma et fibrinogène ainsi que le temps de reptilase, obtenus avec divers dérivés de dextrane (Les pourcentages de motifs B, C, et D, de ces dérivés sont donnés dans le tableau 1).

| Référence (voir Tableau 1) | Concentration initiale (C) du polymère mg/ml | Temps de thrombine : TT sec ± 1 | | Temps de reptilase TR (sec ± 1) sur plasma |
|---|---|---|---|---|
| | | Sur Plasma (Concentration initiale de thrombine : 20 u NIH/ml) Témoin : 8 | Sur Fibrinogène 6 g/l NaCl 0,145 M Témoin 8 | Témoin : 18 |
| 1 (Dextrane) | 50 | 9 | | |
| 2 CM Dextrane | 50 | 8 | | |
| 3 CM Dextrane | 50 | 9 | | |
| 4 | 50 | 9 | | |
| 5 | 50 | 8 | | 18 |
| 6 | 35 | 20 | 22 | 20 |
| 7 | 50 | 18 | 18 | 18 |
| 8 | 40 | 19 | | |
| 9 | 36 | 21 | | |
| 10 | 33 | 18 | | |
| 11 | 20 | 19 | 15 | 18 |
| 12 | 15 | 21 | | |
| 13 | 10 | 18 | | |
| 14 | 6 | 20 | | |
| 15 | 2 | 18 | | |
| 16 | 1,5 | 21 | | |
| 17 | 1,8 | 20 | | |
| 18 | 1,6 | 20 | 9 | 18 |
| 19 | 1,8 | 21 | | |
| 20 | 1,6 | 21 | | |
| 21 | 1,5 | 21 | | |
| 22 | 1,5 | 19 | | |
| 23 | 1,25 | 20 | 8 | 18 |
| 24 | 1 | 22 | 8 | 18 |
| 25 | 1 | 20 | | |
| 26 | 0,8 | 21 | | |
| 27 | 0,3 | 20 | | |
| 28 | 0,3 | 22 | | |
| 29 | 1 | 19 | | |
| 30 | 1 | 23 | | |
| 31 | 0,6 | 20 | | |
| 32 | 0,5 | 22 | | |
| Héparine 173 UI/mg | $6.10^{-3}$ | 18 | | |

TABLEAU 2 : Temps de thrombine (TT) sur plasma et fibrinogène ainsi que temps de reptilase (TR) des dérivés du dextrane.

On constate que les temps de reptilase ne sont pas allongés en présence de polymère et donc le fibrinogène n'est pas altéré. Cette invariabilité des temps de reptilase autorise de corréler l'allongement des temps de thrombine sur plasma à une activité anticoagulante.

Les temps de thrombine sur fibrinogène sont de l'ordre de celui des témoins lorsque les concentrations en dérivés de dextrane sont faibles.

En revanche, lorsque ces concentrations sont élevées on observe un léger allongement de ces temps qui pourrait traduire la possibilité d'une action directe du polymère sur la thrombine. Ces observations témoignent en faveur de la participation d'un autre mécanisme tel que celui dc l'antithrombine III dans ce mécanisme comme dans le cas de l'héparine.

4.2 Activité anticoagulante :

. méthode

L'activité anticoagulante (a) est déterminée à partir des temps de thrombine du PPP en présence de diverses concentrations de polymère.

L'activité a est définie comme étant le nombre d'unités de thrombine inactivées par milligramme de produit (uTh/mg).

Cette quantité de thrombine inactivée est déterminée à partir d'une courbe de calibration.

Dans les mêmes conditions, l'héparine commerciale a un coefficient d'activité a égal à 4000 uTh/mg.

.résultats

On rapporte dans le tableau 3 ci-après les valeurs de l'activité anticoagulante a en uT/mg pour les dérivés considérés dans le tableau 2.

TABLEAU 3

| Référence | Activité anticoagulante a (uT /mg) |
|---|---|
| 1 (Dextrane) | 0 |
| 2 } C.M. Dextrane | 0 |
| 3 } C.M. Dextrane | 0 |
| 4 | 0 |
| 5 | 0 |
| 6 | 1 |
| 7 | 1 |
| 8 | 1 |
| 9 | 1 |
| 10 | 1 |
| 11 | 2 |
| 12 | 3 |
| 13 | 4 |
| 14 | 6 |
| 15 | 14 |
| 16 | 15 |
| 17 | 16 |
| 18 | 17 |
| 19 | 18 |
| 20 | 18 |
| 21 | 20 |
| 22 | 22 |
| 23 | 23 |
| 24 | 29 |
| 25 | 31 |
| 26 | 40 |
| 27 | 65 |
| 28 | 70 |

Variation de l'activité anticoagulante a en fonction des proportions respectives de motifs B et D -

Pour les produits 1 à 28 de l'activité a a été reportée sur les figures 2a et 2b en fonction du taux de motifs B portant les groupes carboxyméthyle ou des motifs D sulfonatés.

La figure 2a correspond à la variation de l'activité a des dérivés de dextrane de Mp de départ de 10 500 en fonction du taux de motifs B, avec un taux de motifs D de 15 ± 1 (courbe ●), de 11 ± 1 (courbe ▲) de 6 ± 1 (courbe △) et de 2 ± 1 (courbe ▽).

La figure 2 b correspond à la variation de l'activité a des dérivés de dextrane utilisés en fonction du taux de motifs D, le taux de motifs B étant de 47,5 ± 2,5%.

L'examen de ces résultats montre qu'on obtient une activité antithrombique lorsque les produits renferment au moins 35% de motifs B.

On note également une augmentation rapide de la valeur a, au-delà du seuil d'apparition de l'activité antithrombotique (figure 2a).

Cette augmentation de a apparaît d'autant plus forte que la proportion de motifs du support dextrane portant des groupes sulfonates est élevée (figure 2b).

Influence du poids moléculaire -

Dans le but d'étudier les corrélations entre masse moléculaire et activité anticoagulante, un dérivé très polydispersé ($\overline{MW}$ = 15600, $\overline{Mn}$ = 7400,

$$\frac{\overline{MW}}{\overline{Mn}} = 2,1),$$

moyennement actif de dextrane (a = 8 uT/mg a été fractionné comme suit :
remplissage de la colonne : on utilise un gel préalablement lavé et mis à gonfler dans de l'eau bidistillée à 20 °C pendant une dizaine d'heures, avant d'être introduit dans une colonne de verre ( LKB) de 1 m de long et 2,5 cm de diamètre intérieur.
éluant : solution aqueuse de NaCl 0,2 M entraînée à 25 ml/h par une pompe à galets "Vario Perpex" (LKB)
charge : 100 mg de produit dans 5ml d'éluant volumes collectés : 8 ml
détection de la concentration de la solution éluée : par spectroscopie UV (280nm).

Après passage sur cette colonne, le produit est fractionné selon sa masse moléculaire en 5 fractions. (Il a été vérifié par dosage chimique que la séparation s'effectuait uniquement en fonction de la masse).

La masse de chaque fraction a été déterminée par chromatographie d'exclusion liquide sous haute pression (90 bars) sur colonne "Merck Lichrospher" 100 diol., en milieu aqueux NaCl 0,2M, et après étalonnage de la colonne.

L'activité anticoagulante a de chaque fraction est résumée dans le tableau 4a ci-après :

TABLEAU 4a

| Fraction | Masse | Activité a (uT /mg) |
|---|---|---|
| 1 | 27 000 | 20 |
| 2 | 19 000 | 14 |
| 3 | 15 000 | 8 |
| 4 | 8 000 | 6 |
| 5 | 6 300 | 3 |

Une opération identique réalisée sur un dextrane ayant un poids moléculaire de l'ordre de 40 000, avec une colonne de 5cm/40cm, un gel Sephadex S 300 Superfine R, en utilisant une charge de 500 mg dans 20 ml permet d'obtenir les 5 fractions identifiées dans le tableau 4 b suivant :

TABLEAU 4b

| Fraction | Masse | Activité a uT/mg |
|---|---|---|
| 1 | 85 000 | 300 |
| 2 | 52 500 | 285 |
| 3 | 39 500 | 270 |
| 4 | 27 500 | 154 |
| 5 | 14 500 | 11 |

Dans le domaine des poids moléculaires considérés on constate que l'activité anticoagulante croît avec lamasse moléculaire.

EXEMPLE 5

Etude de l'action des dérivés de l'invention sur les protéines de la phase contact de la coagulation.

Les protéines relevant de la phase contact sont celles qui s'activent au contact d'une surface autre que l'endothélium et sont responsables, du moins en partie, de l'activation des autres facteurs de la coagulation.

La kallikréine, enzyme issue de l'activation de la phase contact, amplifie l'activation de ce système.

On rapporte ci-après les résultats obtenus avec des dérivés de dextrane selon l'invention du point de vue de leur action sur les protéines de la phase contact.

méthode :

Le test révélateur d'activation de la phase contact consiste à doser l'activité enzymatique de la kallikréine. Celle-ci est mise en évidence par l'amidolyse d'un substrat chromogène spécifique de l'enzyme (substrat S2302 commercialisé par Kabi). La vitesse de libération d'un des produits de l'hydrolyse, la paranitroaniline (pNA) est suivie par spectrophotométrie à 405 nm.

L'augmentation de la densité optique par unité de temps à 405 nm est proportionnelle à l'activité enzymatique de la kallikréine.

La réponse de ce test est globale car elle ne permet pas d'analyser séparément les différentes étapes de l'activation de la phase contact.

L'activation par les dérivés du dextrane a été analysée en fonction de leur concentration et, d'autre part, en fonction de la nature et de leur composition en substituant.

Selon les résultats obtenus l'amplitude de l'activation du système contact apparaît comme une fonction croissante de la concentration en dérivé actif du dextrane.

Le pouvoir activateur des polymères vis-à-vis de la phase contact paraît évoluer en fonction de la nature et de la composition en substituants de manière analogue à leur activité anticoagulante : Une augmentation d'activité apparaît au-delà d'un certain taux de motifs B et semble d'autant plus marquée que le taux de motifs D est élevé.

EXEMPLE 6 :

Etude de l'action des dérivés de dextrane de l'invention sur le système complémentaire

On a étudié l'action de dérivés de dextrane de compositions chimiques variables sur la $C_3$ convertase, alterne, complexe enzymatique capable de cliver la protéine $C_3$, protéine de la voie alterne du système complémentaire.

En présence ou en l'absence d'anticorps spécifiques, le système complémentaire participe aux mécanismes de reconnaissance ou de défense de l'hôte vis-à-vis d'agents infectieux ou de cellules étrangères.

méthode :

Les érythrocytes portant les fragments C3b (petit fragment libéré par le clivage de $C_3$) et appelés plus précisément EA C4b 3b, sont séparés à partir d'érythrocytes de moutons sensibilisés (EA) selon le protocole décrit par M-D-Kazatchkine et coll. J.Cli. Invest. 67, 223,1981.

Les érythrocytes sont ensuite mis en présence de quantités de protéines et stabilisant B, D, P suffisants pour obtenir un site "C3 convertase amplificatrice" par cellule désigné par EA C4b 3b Bb P. Ces sites activent la voie effectrice du complément comprenant les protéines plasmatiques $C_5$-$C_6$-$C_7$-$C_8$-$C_9$ et provoquent l'hémolyse des érythrocytes qui relarguent l'hémoglobine.

Des fractions de 0,1 ml de cette suspension sont prélevées et ajoutées à 0,1 ml de tampon $DGVB^{++}$ - (tampon Véronal + 0,1% de gélatine et contenant 0,15 mM de $Ca^{++}$ et 0,5 mM de $Mg^{++}$, dilué au 1/2 avec une solution de dextrose à 5% contenant 0,15 mM $Ca^{++}$ et 0,5 mM de $Mg^{++}$)seul ou contenant des quantités adéquates de dextrane modifié conforme à la présente invention.

Le mélange est incubé 30 minutes à 30° C sous agitation.

On ajoute 0,3 ml de sérum de rat dilué au 1/20ème dans du tampon GVB-EDTA (tampon Véronal + 0,1% de gélatine et contenant 0,04 M EDTA).

Les sites C3 convertase formés sont révélés par une incubation de 60 minutes à 37° C sous agitation.

On arrête la réaction en ajoutant 1,6 ml de NaCl 0,15M.

Les tubes sont centrifugés et l'intensité de la lyse est déterminée par lecture des surnageants à 412 nm par analyse au spectrophotomètre.

Le nombre de sites hémolytiques par cellule est calculé.

Activité inhibitrice

L'activité inhibitrice du produit testé est exprimée en pourcentage d'inhibition de formation de la convertase par référence au tube témoin où la convertase a été formée en l'absence de dextrane modifié conforme à la présente invention.

Cette activité est exprimée en poids de produit (à volume constant) nécessaire à 50% d'inhibition de formation de la convertase.

Cette activité varie en fonction des motifs.

On rapporte dans le tableau 4 les résultats obtenus avec des dérivés figurant dans les tableaux 1 et 2 ci-dessus en rappelant la composition chimique des produits et leur activité anticoagulante a.

| Référence (N° de l'échantillon cf Tableau 1) | Composition chimique B % | C % | D % | Activité anticomplémentaire µg/$10^7$ EAC 4b 3b Bb P | Activité anticoagulante u.T/mg |
|---|---|---|---|---|---|
| 1 (Dextrane) | 0 | 0 | 0 | ∞ | 0 |
| 3 (CM Dextrane) | 95 | 0 | 0 | 80 | 0 |
| 4 | 51 | 14 | 0 | 60 | 0 |
| 12 | 40 | 12 | 3 | 60 | 3 |
| 16 | 45 | 0 | 4 | 13 | 15 |
| 20 | 67 | 0 | 5 | 11 | 18 |
| 10 | 71 | 7 | 2 | 21,5 | 1 |
| 7 | 37 | 15 | 5 | 60 | 1 |
| 17 | 60 | 14 | 5 | 10 | 16 |
| 6 | 37 | 11 | 9 | 45 | 1 |
| 23 | 43 | 4 | 11 | 7,5 | 23 |
| 22 | 50 | 3 | 10 | 4 | 22 |
| 25 | 68 | 4 | 12 | 4 | 31 |
| 26 | 71,5 | 0 | 10 | 3 | 40 |
| Héparine 159 UI/mg | | | | 1 | 3500 |

L' examen de ces résultats montre que les activités anticomplémentaires des dérivés du dextrane atteignent des valeurs qui sont du même ordre et même pour certains dérivés supérieures à celle de l'héparine.

Sur la figure 3, on a représenté l'évolution de l'activité anticomplémentaire des dérivés du dextrane en fonction de leurs compositions chimiques pour des taux de motifs B variant de 10 à 90% et des taux de motifs D égaux à :

9-12% : courbe u

4-5% : courge v
2-3% : courbe w
0% : O

On constate que l'activité inhibitrice croît en même temps que l'augmentation du taux de motifs D pour un taux de motifs B de 40-50%, l'augmentation du taux des motifs B au-delà de cette valeur n'apportant pas d'amélioration. Il est à noter également que l'évolution en fonction de motifs B et D de l'activité anticomplémentaire est très proche de celle de l'activité anticoagulante a.

On notera que les dérivés à faibles taux de motifs D présentent déjà une activité anticomplémentaire importante alors que leur activité anticoagulante est très faible.

On remarquera, en outre, que la seule présence des motifs C induit une légère activité anticomplémentaire alors qu'elle n'entraîne aucun effet anticoagulant.

Sur la figure 4, on rapporte la courbe obtenue en étudiant la corrélation entre l'activité anticomplémentaire et l'activité anticoagulante des dérivés ci-dessus avec les symboles ■, ▾ ●, et ∇ correspondant respectivement à des dextranes renfermant 9 à 12% de motifs D, 4 à 5 %,0% et 2 à 3%.

Cette figure met en évidence la possibilité d'obtenir des produits dotés d'une forte activité anticomplémentaire et d'une activité anticoagulante faible.

**Revendications**

**1.** Dérivés de dextrane caractérisés en ce qu'il s'agit de produits solubles, possédant un poids moléculaire supérieur à environ 5000 daltons, et qu'ils comportent de manière statistique,

- au moins 35% environ de motifs B constitués de motifs osides substitués par des radicaux possédant une fonction carboxyle répondant à la structure $-O-(CH_2)_n-R-COO^-$ dans laquelle R représente une simple liaison ou un groupe $-CO-NH-(CH_2)_{n'}-$, n est un nombre de 1 à 10 et n'est un nombre de 1 à 7, et
- au moins 3% environ de motifs D, c'est-à-dire des motifs constitués de motifs osidiques de type A, substitués par une chaîne comportant un groupe de structure :

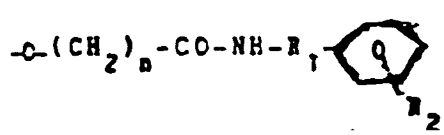

dans laquelle :

- $R_1$ représente une simple liaison, un groupe dans laquelle : $-CH_2-$, ou un groupe

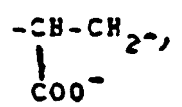

$R_2$ représente un anion d'un sel minéral ou organique physiologiquement tolérable, et n est tel que défini ci-dessus.

**2.** Dérivés selon la revendication 1, caractérisés en ce que $R_2$ représente un groupe $SO_3$.

**3.** Dérivés de dextrane selon les revendications 1 ou 2, caractérisés en ce qu'ils renferment, en outre, des motifs C substitués par des radicaux de structure

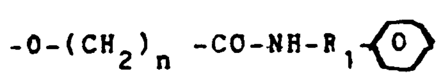

dans laquelle $R_1$ et n sont tels que définis ci-dessus.

**4.** Dérivés de dextrane selon la revendication 3, caraecérisés en ce que,l'ensemble des motifs A non substitués et des motifs C représente au plus 60% du nombre total de motifs.

**5.** Dérivés de dextrane selon l'une quelconque des revendications 1 à 4, caractérisés en ce qu'ils comprennent des motifs B substitués par une chaîne $-O-CH_2-COO^-$, ou des groupes carboxyéthyle, carboxypropyle ou carboxybutyle $-O-(CH_2)_n-COO^-$, n étant égal respectivement à 2,3 ou 4, ou des groupes $-O-(CH_2)_n-CO-NH-(CH_2)_{n'}-COO^-$, n' étant égal à 4,5 ou 7.

**6.** Dérivés de dextrane selon l'une quelconque des revendications 1 à 5, caractérisés en ce qu'ils renferment des motifs D substitués par une chaîne de structure

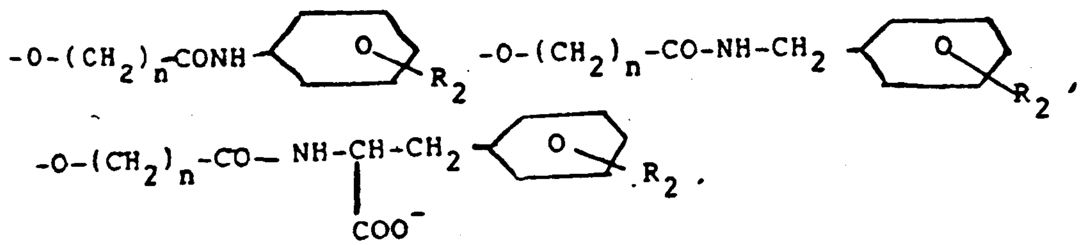

$R_2$ étant tel que défini ci-dessus et représentant avantageusement un groupe $-SO_3^-$ et n étant un nombre de 1 à 4.

**7.** Dérivés de dextrane selon l'une quelconque des revendications 1 à 6, caractérisés en ce qu'ils possèdent des poids moléculaires élevés supérieurs à 40000 environ et renfermant de 40 à 50% de motifs B.

**8.** Dérivés de dextrane selon l'une quelconque des revendications 1 à 6, caractérisés en ce qu'ils renferment de 40 à 50% environ de motifs B et de 3 à 4% environ de motifs D et qu'ils possèdent des poids moléculaires de l'ordre de 10 000.

**9.** Dérivés selon la revendication 1, caractérisés en ce qu'ils comprennent les motifs A,B,C et D suivants :

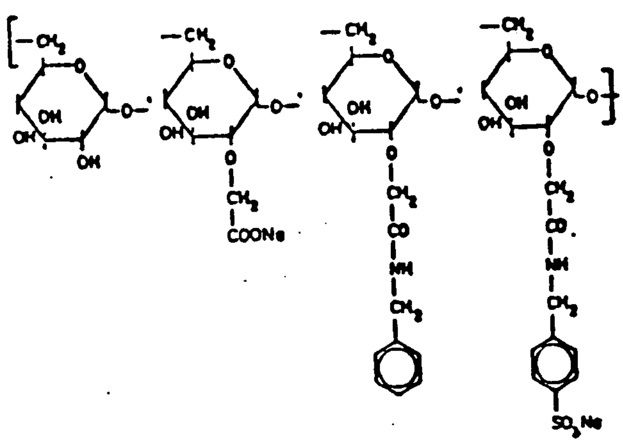

A B C D

selon les proportions définies dans les revendications précédentes.

**10.** Procédé de préparation des dérivés de dextrane selon la revendication 1, caractérisé en ce qu'il comprend

- la réaction d'un dextrane avec un dérivé de formule :

  $X(CH_2)_n$-R-COOH

  dans laquelle X représente un groupe réactif capable d'établir une liaison de glucosylation avec

un groupe -OH d'un motif glucosyle, ce qui conduit à la formation de motifs B;

- la réaction du dextrane renfermant les motifs A et B avec un dérivé de formule

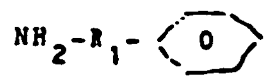

dans laquelle $R_1$ est tel que défini ci-dessus afin d'obtenir la fixation par un pont amide du groupe aryle substitué lu radical provenant des chaînes de substitution des motifs D, ce qui permet d'introduire des motifs C sur la chaîne,

- l'introduction de groupes anioniques, sur les groupes aryle des motifs C, pour obtenir les motifs D,
- le fractionnement éventuel des dérivés de dextrane afin d'éliminer les dérivés présentant un poids moléculaire inférieur à 5000.

**11.** Procédé selon la revendication 10, caractérisé en ce que les groupes anioniques introduits sont des groupes $SO_3^-$.

**12.** Procédé selon la revendication 10, caractérisé en ce que

- pour l'élaboration de motifs B, on fait avantageusement réagir avec le dextrane un dérivé réactif tel qu'un halogénure, notamment un chlorure, en opérant en milieu basique, à une température de l'ordre de 0°C puis en portant la température à 70°C environ ou, en variante, en procédant selon un gradient de température de l'ambiante à 55°C environ, le rapport de la concentration en dérivé réactif à celle du dextrane étant avantageusement de 1,5 à 3,5 environ, et qu'on récupère les produits par précipitation à l'aide d'un solvant alcoolique tel que l'éthanol et en abaissant le pH à un pH neutre.

**13.** Procédé selon les revendications 10 ou 12 caractérisé en ce que pour l'élaboration des motifs C, on effectue un couplage des substitutions des motifs B avec un composé de formule

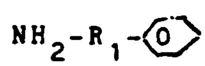 ,

en présence d'un agent de couplage usuel, en milieu acide, à température ambiante, ou, en variante,on prépare l'anhydride mixte du dextrane en faisant réagir un chloroformate d'alcoyle et un dérivé capable de former un chlorhydate avec HCl libéré, tel que la N-méthylmopholine, la triéthylamine ou analogue, en opérant à une température inférieure à 0°C environ , puis, on réalise le couplage à basse température, le rapport de la concentration en dérivé à coupler à celle du dextrane substitué étant avantageusement de l'ordre de 2 et celui du chloroformate au dextrane d'environ 1.

**14.** Substituts de plasma ou expandeurs, caractérisés en ce qu'ils sont élaborés à partir d'un dérivé de dextrane selon l'une quelconque des revendications 1 à 8.

## Claims

**1.** Dextran derivatives, characterised in that they are soluble products having a molecular weight greater than about 5000 daltons and that, statistically, they comprise

- at least about 35% of B factors consisting of oside factors substituted by radicals having a carboxyl function corresponding to the structure $-O-(CH_2)_n-R-COO^-$ wherein R represents a single bond or a $-CO-NH-(CH_2)_{n'}$-group, n is a number from 1 to 10 and n' is a number from 1 to 7, and
- at least about 3% of D factors, i.e. factors consisting of oside factors of type A substituted by a chain comprising a group of the structure:

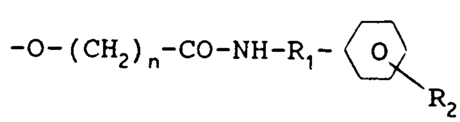

wherein

- $R_1$ represents a single bond, a group $-CH_2-$ or a group

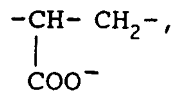

$R_2$ represents an anion of a physiologically acceptable organic or inorganic salt, and n is as hereinbefore defined.

**2.** Derivatives according to claim 1, characterised in that $R_2$ represents a group $SO^-_3$.

**3.** Dextran derivatives according to claim 1 or 2, characterised in that they further contain C factors substituted by radicals of the structure

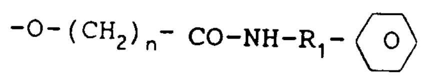

wherein $R_1$ and n are as hereinbefore defined.

**4.** Dextran derivatives according to claim 3, characterised in that all the unsubstituted A factors and C factors constitute at most 60% of the total number of factors.

**5.** Dextran derivatives according to any of claims 1 to 4, characterised in that they comprise B factors substituted by an $-O-CH_2-COO^-$ chain, or carboxyethyl, carboxypropyl or carboxybutyl groups $-O-(CH_2)_n-COO^-$, wherein n represents 2, 3 or 4, respectively, or groups of the formula $-O-(CH_2)_n-CO-NH-(CH_2)_{n'}-COO^-$, wherein n' represents 4, 5 or 7.

**6.** Dextran derivatives according to any one of claims 1 to 5, characterised in that they contain D factors substituted by a chain of the structure

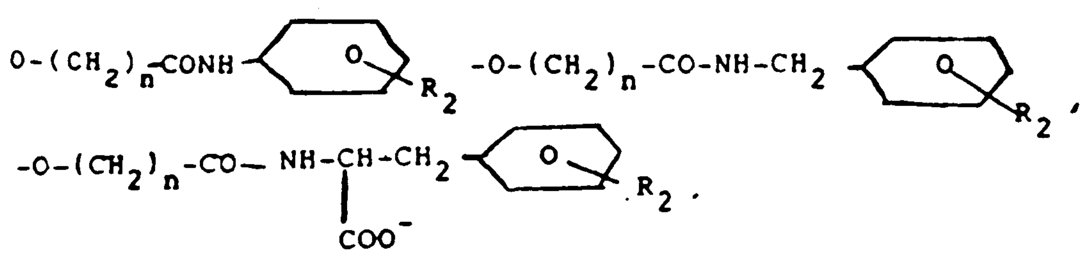

$R_2$ being defined as hereinbefore and advantageously representing a group $-SO_3^-$ and n being a number from 1 to 4.

**7.** Dextran derivatives according to any one of claims 1 to 6, characterised in that they have high molecular weights, greater than about 40,000, and contain 40 to 50% of B factors.

**8.** Dextran derivatives according to any one of claims 1 to 6, characterised in that they contain about 40 to 50% of B factors and about 3 to 4% of D factors and that they have molecular weights of the order of 10,000.

**9.** Derivatives according to claim 1, characterised in that they contain the following A, B, C and D factors:

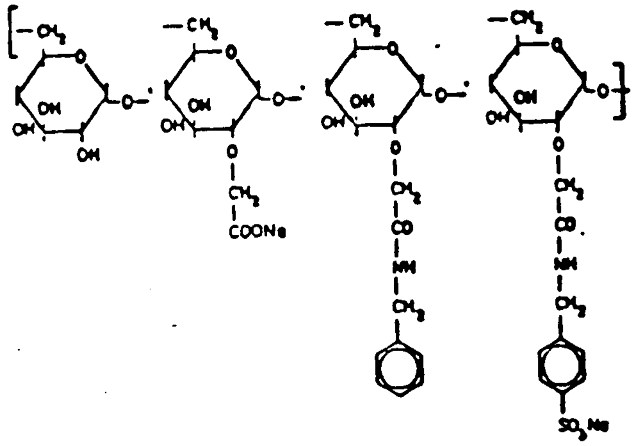

A B C D

in the proportions defined in the preceding claims.

**10.** A process for preparing the dextran derivatives according to claim 1, characterised in that it comprises

- reacting a dextran with a derivative of the formula:

  $X(CH_2)_n$-R-COOH

  wherein X represents a reactive group capable of forming a glucosylation bond with an -OH group of a glucosyl factor, resulting in the formation of B factors;
- reacting dextran containing A and B factors with a derivative of formula

  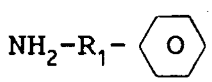

  wherein $R_1$ is as hereinbefore defined, in order to achieve fixing by an amide bridge of the substituted aryl group to the radical originating from the substitution chains of the D factors, thus enabling C factors to be introduced into the chain,
- introducing anionic groups into the aryl groups of the C factors in order to obtain D factors,
- possible fractionation of the dextran derivatives to eliminate derivatives having a molecular weight of less than 5000.

**11.** A process according to claim 10, characterised in that the anionic groups introduced are $SO_3^-$groups.

**12.** Process according to claim 10, characterised in that

- in order to prepare B factors, dextran is advantageously reacted with a reactive derivative such as a halide, especially a chloride, in a basic medium, at a temperature of the order of 0 ° C, then raising the temperature to about 70 ° C or, alternatively, by proceeding along a temperature gradient from ambient temperature to about 55 ° C, the ratio of the concentration of reactive derivative to that of dextran advantageously being from about 1.5 to 3.5, and the products are recovered by precipitation using an alcoholic solvent such as ethanol and by lowering the pH to a neutral pH.

**13.** A process according to claim 10 or 12, characterised in that, in order to prepare C factors, the substituents of the B factors are coupled with a compound of formula

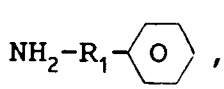

in the presence of a conventional coupling agent, in an acid medium, at ambient temperature, or, alternatively, the mixed anhydride of dextran is prepared by reacting an alkyl chloroformate and a derivative capable of forming a hydrochloride with liberated HCl, such as N-methylmorpholine, triethylamine or the like, working at a temperature of less than about 0 ° C, then the coupling is carried out at low temperature, whilst the ratio of the concentration of derivative to be coupled to the concentration of substituted dextran is advantageously of the order of 2 and the ratio of chloroformate to dextran is approximately 1.

**14.** Plasma substitutes or expanders, characterised in that they are developed from a dextran derivative according to any one of claims 1 to 8.

**Patentansprüche**

**1.** Dextranderivate,
dadurch gekennzeichnet, daß sie löslich sind, eine Molekülmasse über etwa 5000 besitzen und statistisch verteilt enthalten,

- mindestens etwa 35 % Struktureinheiten B, die aus substituierten glucosidischen Einheiten bestehen, wobei die Substituenten eine Carboxylfunktion mit der Struktur $-O-(CH_2)_n-R-COO^-$ aufweisen, worin R eine Einfachbindung oder eine Gruppe $-CO-NH-(CH_2)_{n'}-$, wobei n' ein Zahl von 1 bis 7 darstellt, und n eine Zahl von 1 bis 10 bedeuten,
- mindestens etwa 3 % Struktureinheiten D, die Struktureinheiten aus glucosidischen Einheiten des Typs A darstellen, die mit einer Kette substituiert sind, die eine Gruppe der Struktur

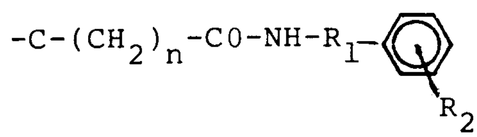

enthält, worin $R_1$ eine Einfachbindung, eine Gruppe $-CH_2$-oder eine Gruppe

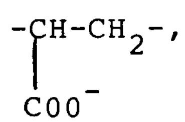

$R_2$ ein Anion eines anorganischen oder organischen physiologisch verträglichen Salzes und n das gleiche wie oben bedeuten.

**2.** Derivate nach Anspruch 1,
dadurch gekennzeichnet,
daß $R_2$ eine $SO_3^-$ -Gruppe ist.

**3.** Dextranderivate nach Anspruch 1 oder 2,
dadurch gekennzeichnet,
daß sie ferner Struktureinheiten C enthalten, die mit Substituenten der Struktur

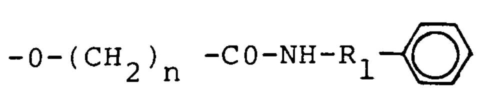

substituiert sind, worin $R_1$ und n die gleiche Bedeutung wie oben haben.

**4.** Dextranderivate nach Anspruch 3,
dadurch gekennzeichnet,

daß die nicht substituierten Struktureinheiten A und die Struktureinheiten C zusammen höchstens 60 % der Gesamtzahl der Struktureinheiten ausmachen.

5. Dextranderivate nach einem der Ansprüche 1 bis 4, dadurch gekennzeichnet, daß sie Struktureinheiten B enthalten, die substituiert sind mit $-O-CH_2-COO^-$, Carboxyethyl, Carboxypropyl oder Carboxybutyl, $-O-(CH_2)_n-COO^-$, wobei n gleich 2, 3 oder 4 ist, oder mit $-O-(CH_2)_n-CO-NH-(CH_2)_{n'}-COO^-$, wobei n' gleich 4, 5 oder 7 ist.

6. Dextranderivate nach einem der Ansprüche 1 bis 5, dadurch gekennzeichnet, daß sie Struktureinheiten D enthalten, die mit einer Kette der Struktur

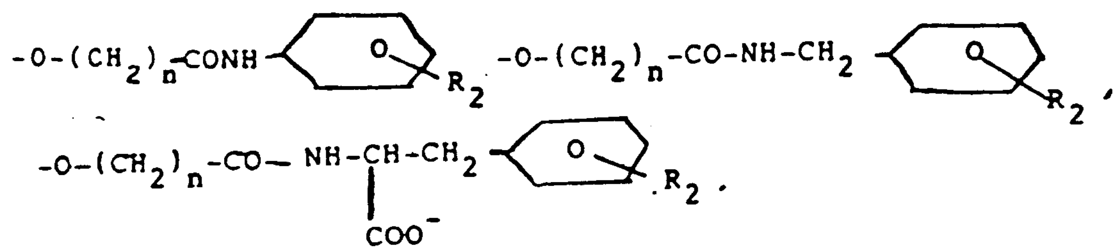

substituiert sind, wobei $R_2$ das gleiche wie oben und vorteilhaft eine $SO_3^-$-Gruppe und n eine Zahl von 1 bis 4 bedeuten.

7. Dextranderivate nach einem der Ansprüche 1 bis 6, dadurch gekennzeichnet, daß sie eine Molekülmasse über etwa 40000 besitzen und 40 bis 50 % Struktureinheiten B enthalten.

8. Dextranderivate nach einem der Ansprüche 1 bis 6, dadurch gekennzeichnet, daß sie etwa 40 bis 50 % Struktureinheiten B und etwa 3 bis 4 % Struktureinheiten D enthalten und Molekülmassen in der Größenordnung von 10000 besitzen.

9. Derivate nach Anspruch 1, dadurch gekennzeichnet, daß sie die folgenden Struktureinheiten A, B, C und D in gemäß den vorhergehenden Ansprüchen festgelegten Anteilen enthalten:

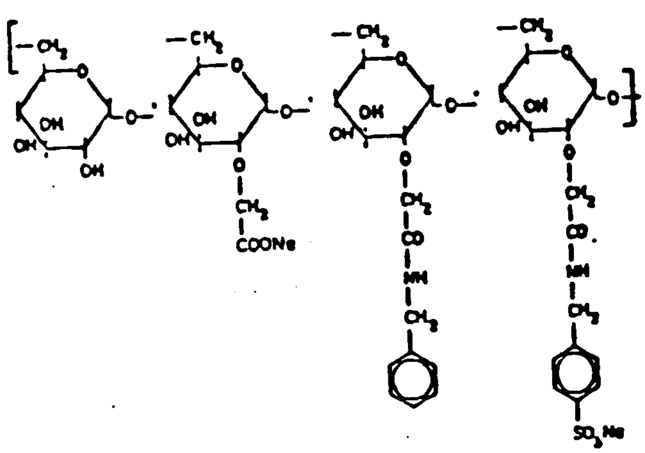

A B C D

**10.** Verfahren zur Herstellung der Dextranderivate nach Anspruch 1,
dadurch gekennzeichnet,
daß es die folgenden Stufen umfaßt:

- Umsetzung eines Dextrans mit einem Derivat der Formel

  $X(CH_2)_n\text{-}R\text{-}COOH$ ,

  worin X eine reaktive Gruppe ist, die zur Bildung einer glucosidischen Bindung mit einer -OH-Gruppe einer Glucosyl-Struktureinheit befähigt ist, unter Erhalt von Struktureinheiten B,
- Umsetzung des die Struktureinheiten A und B enthaltenden Dextrans mit einem Derivat der Formel

  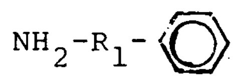 ,

  worin $R_1$ das gleiche wie oben bedeutet, zur Verknüpfung der substituierten Arylgruppe über eine Amidbindung mit den Substituenten der Struktureinheiten B, was die Einführung der Struktureinheiten C in die Kette ermöglicht,
- Einführung anionischer Gruppen in die Arylgruppen der Struktureinheiten C unter Erhalt von Struktureinheiten D, sowie
- gegebenenfalls Fraktionierung der Dextranderivate zur Abtrennung von Derivaten mit einer Molekülmasse unter 5000.

**11.** Verfahren nach Anspruch 10,
dadurch gekennzeichnet,
daß die eingeführten anionischen Gruppen $SO_3^-$ -Gruppen sind.

**12.** Verfahren nach Anspruch 10,
dadurch gekennzeichnet,
daß man für die Herstellung der Struktureinheiten B vorteilhaft das Dextran mit einem reaktiven Derivat, wie einen Halogenid, insbesondere einem Chlorid, in basischem Milieu bei einer Temperatur der Größenordnung von 0 °C, wobei man anschließend die Temperatur auf ungefähr 70 °C hält, umsetzt, oder indem man alternativ bei einem Temperaturgradienten von Umgebungstemperatur bis etwa 55 °C arbeitet, wobei das Verhältnis der Konzentration des reaktiven Derivats zu der des Dextrans vorteilhaft 1,5 bis 3,5 beträgt, und daß man die Produkte durch Ausfällung mit Hilfe eines alkoholischen Lösungsmittels wie Ethanol unter Absenken des pH-Werts auf einen neutralen pH-Wert isoliert.

**13.** Verfahren nach den Ansprüchen 10 oder 12,
dadurch gekennzeichnet,
daß man für die Herstellung der Struktureinheiten C eine Verknüpfung der Substituenten der Struktureinheiten B mit einer Verbindung der Formel

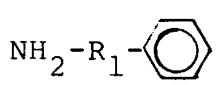

in Gegenwart eines gebräuchlichen Kupplungsmittels in saurem Medium bei Umgebungstemperatur durchführt, oder man alternativ das gemischte Anhydrid des Dextrans herstellt, indem man ein Alkylchlorformiat mit einem zur Bildung eines Hydrochlorids mit dem freigesetzen Chlorwasserstoff befähigten Derivat, wie N-Methylmorpholin, Triethylamin oder analogen Verbindungen umsetzt, wobei die Temperatur unter etwa 0 °C liegt, und man anschließend die Kupplung bei niedriger Temperatur durchführt, wobei das Verhältnis der Konzentration des anzukuppelnden Derivats und der des substituierten Dextrans vorteilhaft in der Größenordnung von 2 und das des Chlorformiats zum Dextran in der Größenordnung von 1 liegen.

**14.** Plasmaersatz oder Plasmaexpander, dadurch gekennzeichnet, daß sie ausgehend von einem Dextranderivat nach einem der Ansprüche 1 bis 8 hergestellt sind.

FIG. 1.

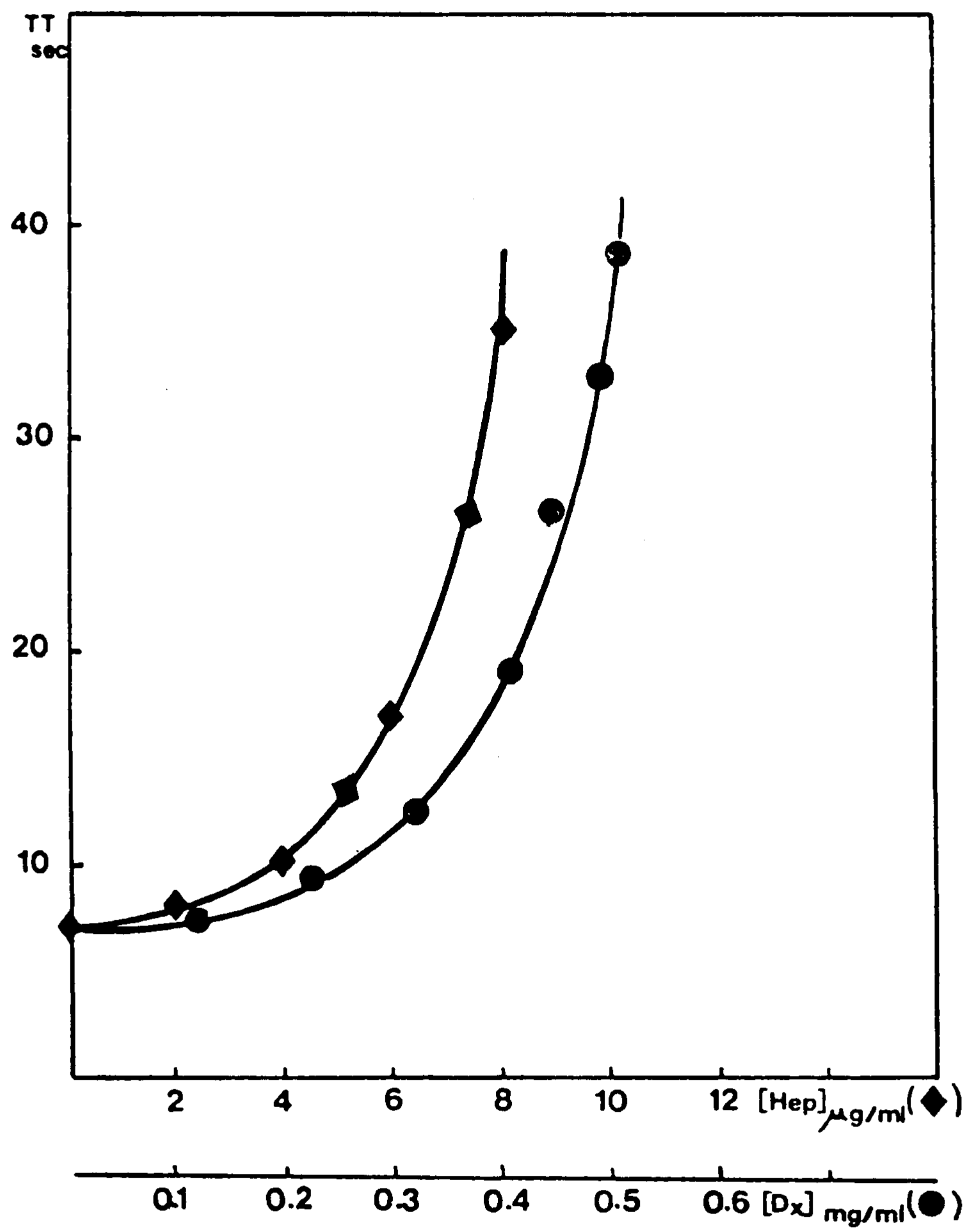

FIG.2a.

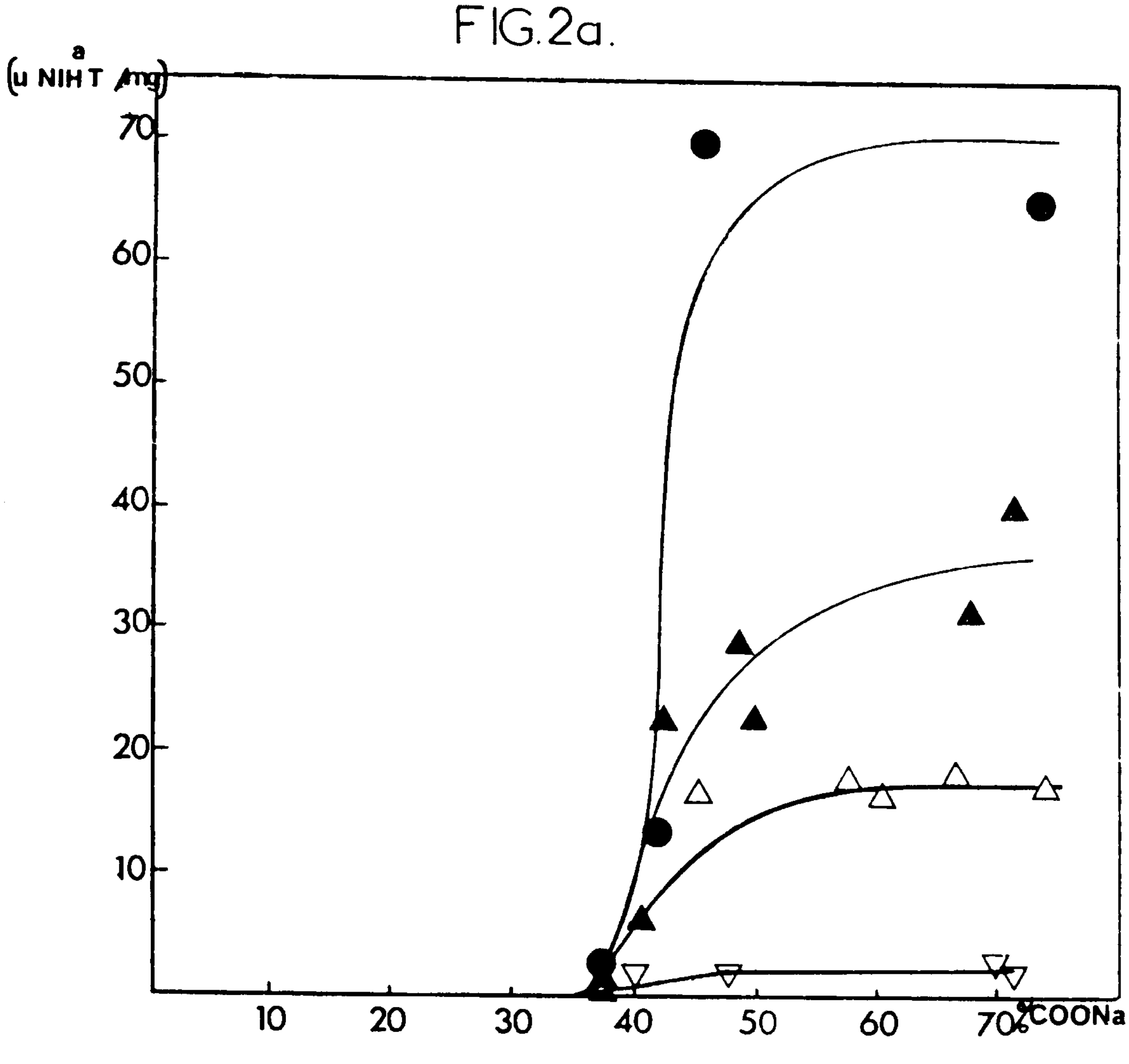
(u NIH$^{a}$T /mg)
70
60
50
40
30
20
10
10
20
30
40
50
60
70
%COONa

FIG. 2b.

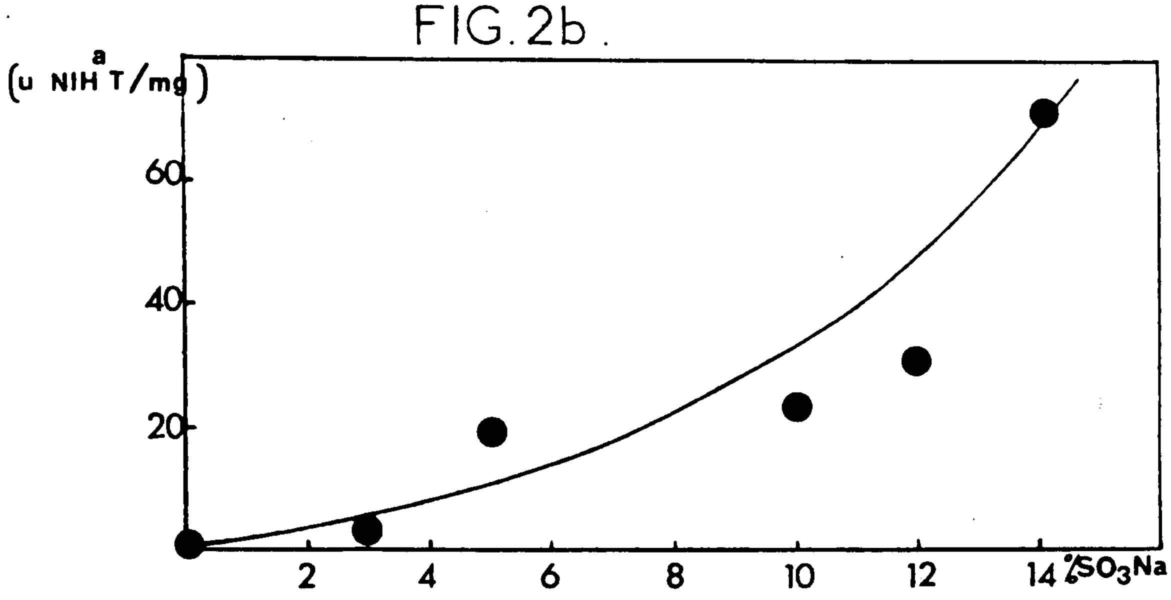
(u NIH$^{a}$T/mg)
60
40
20
2
4
6
8
10
12
14
%SO3Na

FIG.3.

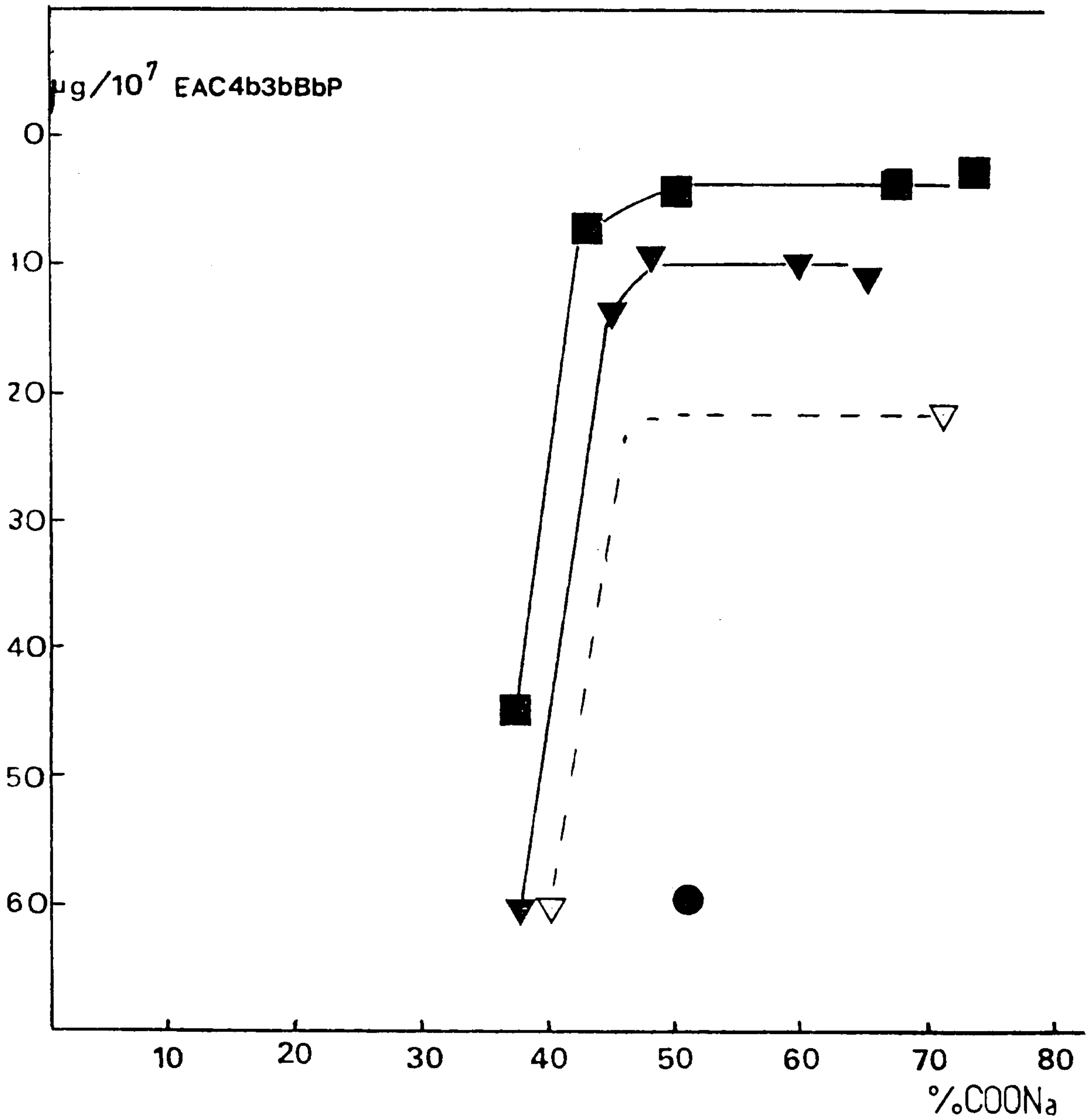

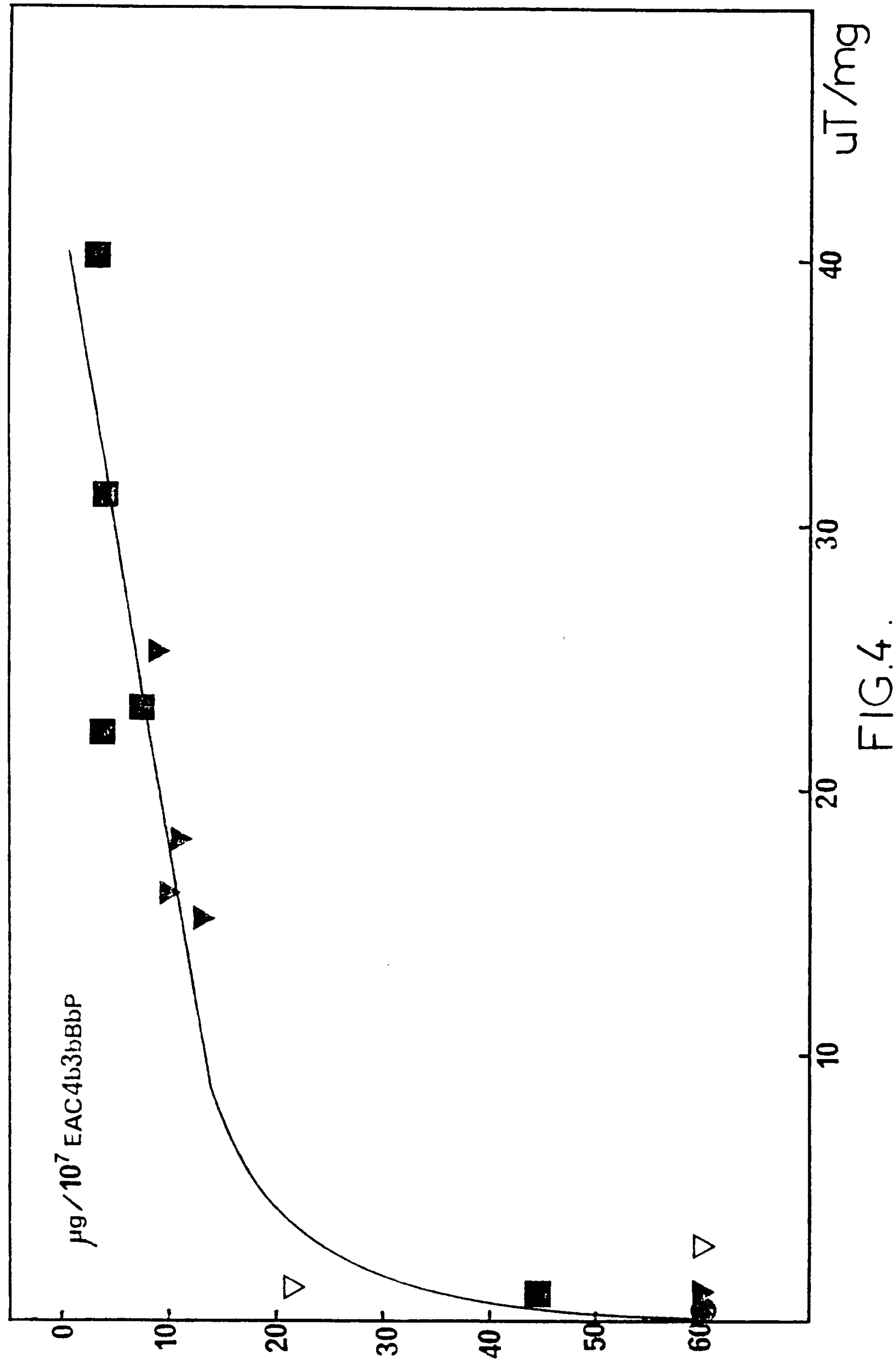


FIG.4.